# EUROPÄISCHE PATENTSCHRIFT

(11) **EP 1 930 316 B1**
(45) Veröffentlichungstag und Bekanntmachung des Hinweises auf die Patenterteilung: **08.08.2012**
(21) Anmeldenummer: 07121136.1
(22) Anmeldetag: 20.11.2007
(51) Int. Cl.: C07C 253/14, C07D 213/61

(54) **Verfahren zur katalytischen Herstellung von aromatischen oder heteroaromatischen Nitrilen**
Method for catalytic manufacture of aromatic or heteroaromatic nitriles
Procédé destiné à la fabrication catalytique de nitriles aromatiques ou hétéroaromatiques

(30) Priorität: 29.11.2006 DE 102006056208
(43) Veröffentlichungstag der Anmeldung: 11.06.2008
(73) Patentinhaber: Saltigo GmbH, 40764 Langenfeld (DE)
(72) Erfinder: Müller, Nikolaus, 40789, Monheim (DE); Mägerlein, Wolfgang, 68165, Mannheim (DE); Cotté, Alain, 51377, Leverkusen (DE); Beller, Matthias, 18211, Ostseebad Nienhagen (DE); Schareina, Thomas, 18195, Cammin (DE); Zapf, Alexander, 83024, Rosenheim (DE)
(74) Vertreter: Pettrich, Klaus-Günter

(56) Entgegenhaltungen:
- DATABASE MEDLINE [Online] US NATIONAL LIBRARY OF MEDICINE (NLM), BETHESDA, MD, US; 21. Juni 2004 (2004-06-21), SCHAREINA THOMAS ET AL: "Potassium hexacyanoferrate(II)--a new cyanating agent for the palladium-catalyzed cyanation of aryl halides." XP002469910 Database accession no. NLM15179478 -& CHEMICAL COMMUNICATIONS (CAMBRIDGE, ENGLAND) 21 JUN 2004, Nr. 12, 21. Juni 2004 (2004-06-21), Seiten 1388-1389, XP002469909 ISSN: 1359-7345
- CHENG Y-N ET AL: "Cyanation of aryl chlorides with potassium hexacyanoferrate(II) catalyzed by cyclopalladated ferrocenylimine tricyclohexylphosphine complexes" SYNLETT 01 MAR 2007 GERMANY, Nr. 4, 1. März 2007 (2007-03-01), Seiten 543-546, XP002469629 ISSN: 0936-5214
- SCHAREINA ET AL: "A new palladium catalyst system for the cyanation of aryl chlorides with K4[Fe(CN)6]" TETRAHEDRON LETTERS, ELSEVIER, AMSTERDAM, NL, Bd. 48, Nr. 7, 17. Januar 2007 (2007-01-17), Seiten 1087-1090, XP005834095 ISSN: 0040-4039
- BEDFORD R B ET AL: "The development of palladium catalysts for CC and Cheteroatom bond forming reactions of aryl chloride substrates" COORDINATION CHEMISTRY REVIEWS, ELSEVIER SCIENCE, AMSTERDAM, NL, Bd. 248, Nr. 21-24, Dezember 2004 (2004-12), Seiten 2283-2321, XP004917431 ISSN: 0010-8545

## Beschreibung

Die vorliegende Erfindung betrifft ein Verfahren zur Herstellung von ggf. substituierten aromatischen oder heteroaromatischen Nitrilen durch Cyanierung der entsprechenden Arylhalogenide mit Katalysatoren auf Basis von Palladium-Komplexen unter Verwendung von gelbem Blutlaugensalz als Cyanidquelle.

Aromatische oder heteroaromatische Nitrile haben technische Bedeutung als Ausgangsprodukte für Feinchemikalien, Agroprodukte und Pharmazwischenprodukte. Eine technisch angewandte Methode zur Herstellung von aromatischen Nitrilen ist die Ammonoxidation von substituierten Toluolen. Dieses Verfahren ist industriell nur interessant, wenn die entsprechenden Toluole kostengünstig verfügbar sind. Darüber hinaus gelingt die Ammonoxidation nicht in Gegenwart von oxidationsempfindlichen Substituenten im Substrat. Weitere technische Verfahren zur Darstellung von Benzonitrilen sind Umsetzungen von Carbonsäuren und Ammoniumsalzen oder Amiden durch Destillation mit stark wasserbindenden Substanzen (z. B. P₂O₅) sowie Reaktion von Carbonsäuren oder Estern in der Dampfphase mit Ammoniak über einem Al-Festbett bei 500°C.

Kostengünstige Ausgangsmaterialien für aromatische Nitrile stellen die entsprechenden Chlorbenzole und teilweise die entsprechenden Brombenzole dar. Allerdings gelingt die Substitution von Chlor mit Cyanid nach bekannten Verfahren oft nur unbefriedigend. Beispielsweise reagieren aromatische Halogenide mit HCN in der Dampfphase bei 650 °C oder bei 480 - 650 °C in Anwesenheit eines Metall- oder Metalloxidkatalysators. Katalysatoren, die die Umsetzung von Arylhalogeniden mit Cyanid bei milderen Reaktionsbedingungen beschleunigen, sind Palladium- und Nickelkomplexe. So beschreiben R. Breitschuh, B. Pugin, A. Idolese und V. Gisin (EP-A 0 787 124 B1 und US-A 5,883,283) die Darstellung von subst. 3-Aminobenzonitrilen aus den entsprechenden subst. 3-Aminochlorbenzolen in Gegenwart von bevorzugt Ni-Komplexen und stöchiometrischen Mengen einer komplexierenden Substanz. Nachteilig bei diesem Verfahren ist die Verwendung von einem Überschuss an Reduktionsmittel und die Beschränkung der Reaktion auf eine spezielle Substratklasse.

B. R. Cotter (US-A 4,211,721) beschreibt den positiven Einfluss von Etherkomponenten aus der Gruppe 18-Krone-6, Polyether, Alkoxypolyether oder Mixturen dieser mit einer Molmasse 200 - 25.000 als Co-Katalysator auf die palladiumkatalysierte Cyanierung von Arylhalogeniden. In den Beispielen der genannten Anmeldung wird jedoch deutlich, dass unter den erfindungsgemäßen Reaktionsbedingungen lediglich aktivierte (elektronenarme) Chloraromaten wie 4-Chlorbenzotrifluorid reagieren, wobei die Produktausbeuten an den entsprechenden Benzonitrilen bei nur ca. 45 % liegen. Nichtaktivierte Chloraromaten wie Chlortoluol ergeben das Zielprodukt in nur 5 bis 11 % Ausbeuten. Derartige Ausbeuten machen eine industrielle Umsetzung des Verfahrens wirtschaftlich unmöglich.

J. B. Davison, R. J. Jasinski und P. J. Peerce-Landers (US-A 4,499,025) beschreiben die Herstellung aromatischer Nitrile aus Chloraromaten, katalysiert durch einen Gruppe-VIII-Metall(0)komplex, welcher elektrochemisch gebildet wird. Diese Verfahrensweise ist jedoch im Vergleich zu herkömmlichen Batch-Verfahren außerordentlich teuer. Darüber hinaus gibt es kein Beispiel für die erfolgreiche Umsetzung eines Chloraromaten in guten Ausbeuten.

A. Viauvy und M. Casado (EP-A 0 994 099 Al) beschreiben weiterhin die Umsetzung von Chloraromaten zum entsprechenden Nitril mit Kupfercyanid und einer Bromidquelle oder Alkalimetallcyanid bzw. Tetraalkylanunoniumcyanid in Anwesenheit von Kupferbromid und einem Phasentransferkatalysator oder Kupfercyanid und Lithiumiodid. Diese Verfahrensweisen haben den Nachteil, dass überstöchiometrische Mengen an Schwernemetallsalzabfällen gebildet werden. Weiterhin sind die Ausbeuten an Benzonitrilen aus Chloraromaten nicht befriedigend.

M.-H. Rock und A. Merhold (DE-A 197 06 648 A1 und WO 98/37 058) beschreiben die Herstellung aromatischer Nitrile aus Chloraromaten in Gegenwart eines Nickelkatalysators und eines Ketons durch Umsetzung mit Cyaniden. Die Reaktion kann jedoch nur dann erfolgreich durchgeführt werden, wenn die Cyanidkonzentration genau kontrolliert wird, da ansonsten der Katalysator irreversibel cyaniert wird. Nachteilig bei diesem Verfahren ist wiederum der notwendige Zusatz eines Reduktionsmittels wie Zink, der zu zusätzlichen Schwermetallsalzabfällen führt und die Verwendung von speziellen Ketonen als Lösemittel.

R. K. Arvela und N. E. Leadbeater beschreiben die Cyanierung von Chloraromaten in Gegenwart stöchiometrischer Mengen Nickel(II)bromid und Natriumcyanid (J. Org. Chem. 2003, 68, 9122-5). Neben der daraus resultierenden Menge an Nickelsalzabfällen ist die Verwendung von Mikrowellenstrahlung zum Energieeintrag für technische Umsetzungen nachteilig.

H. R. Chobanian, B. P. Fors und L. S. Lin beschreiben die Umsetzung von Chloraromaten mit Zink(II)cyanid unter Verwendung eines Pd-Katalysators mit S-Phos als Liganden (Tetrahedron Lett. 2006, 47, 3303-5). Nachteilig ist auch hier die Generierung stöchiometrischer Mengen an Schwerznetallsalzabfällen sowie der Einsatz eines teuren Phosphan-Liganden in hoher Konzentration (2-10 mol-% bezogen auf den Chloraromaten).

Beller und Mitarbeiter beschreiben den Einfluss von Kronenethern, Diphosphinliganden und Diaminliganden auf die palladiumkatalysierte Umsetzung von Arylhalogeniden mit Alkalicyaniden (DE-A 101 13 976, Tetrahedron Lett. 2001, 42, 6707-10). Auf diesen Arbeiten fußend wurde die dosierte Zugabe von Acetoncyanhydrin als Cyaniddonor im beschriebenen System getestet (Angew. Chem. 2003, 115, 1700-3). Weiterhin wurde die Dosierung von TMSCN (J. Organomet. Chem. 2003, 684, 50-5) bzw. Blausäure (DE-A 103 23 574) beschrieben.

Alle vorstehend beschriebenen Verfahren zur Cyanierung von Aryl- oder Heteroarylhalogeniden haben den Nachteil, dass sie Cyanidquellen verwenden, bei denen freies Cyanid im Überschuss vorliegt, so dass der Cyanidligand aufgrund seiner stark komplexierenden Wirkung den Pd-Katalysator blockieren kann. Daher weisen diese Verfahren generell den Nachteil auf, dass die Cyanidquelle kontrolliert dosiert werden muss und/oder dass oft schlechte Katalysatoraktivitäten und produktivitäten beobachtet werden. Zudem sind diese Cyanidquellen hochtoxisch und können leicht Blausäure freisetzen, so dass ihr technischer Einsatz nur unter besonderen Sicherheitsmaßnahmen möglich ist.

Diese Nachteile wurden von Beller et al. dadurch umgangen, dass gelbes Blutlaugensalz, Kaliumhexacyanoferrat(II) K₄[Fe(CN)₆], als ungiftige und leicht handhabbare Cyanidquelle eingesetzt wurde. Dieses Cyanierungsreagenz ist wenig giftig, löst sich in Wasser ohne Zersetzung und wird sogar in der Lebensmittelindustrie z.B. bei der Herstellung von Tafelsalz oder zum Schonen von Weinen eingesetzt (Roempp Lexikon Chemie, Georg Thieme Verlag, Stuttgart/New York, 1999). In Chem. Commun. 2004, 1388-1389 wird ein Pd-katalysiertes Verfahren zur Cyanierung unter Verwendung von gelbem Blutlaugensalz beschrieben. In den Anmeldungen DE-A 10 2005 009 517.8 und DE-A 10 2006 042439.5 werden Cu-katalysierte Verfahren zur Cyanierung unter Verwendung von gelbem Blutlaugensalz, K₄[Fe(CN)₆], oder rotem Blutlaugensalz, K₃[Fe(CN)₆], beschrieben. Nachteilig ist jedoch, dass mit diesen Verfahren nur Aryl- und Heteroarylbromide effizient umgesetzt werden können. Für technische Anwendungen sind jedoch Aryl- und Heteroarylchloride wesentlich attraktiver, da sie im allgemeinen kostengünstiger sowie breiter verfügbar und zugänglich sind als andere Aryl- oder Heteroarylhalogenide oder pseudohalogenide. Mit den bisher aus dem Stand der Technik bekannten Verfahren, die Blutlaugensalze zur Cyanierung einsetzen, ist jedoch eine Umsetzung der im Vergleich zu Aryl- oder Heteroarylbromiden reaktionsträgeren Aryl- oder Heteroarylchloriden nicht oder nur mit sehr geringen Ausbeuten möglich. Dies trifft insbesondere für nicht aktivierte oder deaktivierte (elektronenreiche) sowie sterisch gehinderte Aryl- bzw. Heteroarylchloride zu. Als einziges Beispiel einer erfolgreichen Umsetzung wurde oben zitierter Chem. Commun.-Veröffentlichung die Pd-katalysierte Cyanierung des stark aktivierten 4-Chlorchinolins unter Verwendung von gelbem Blutlaugensalz beschrieben.

Aufgabe der vorliegenden Erfindung war somit die Entwicklung eines Verfahrens zur Cyanierung von Aryl- und Heteroarylhalogeniden mit Blutlaugensalz, das sich gegenüber dem Stand der Technik insbesondere durch eine größere Substratbreite, insbesondere in Bezug auf eine effiziente Umsetzung von sowohl aktivierten als auch deaktivierten Aryl- und Heteroary1chloriden auszeichnet. Ebenso sollte das erfindungsgemäße Verfahren im technischem Maßstab gut einsetzbar sein und den Verfahren des Standes der Technik in Bezug auf Katalysatorproduktivität und damit auch in Bezug auf ökonomische Gesichtspunkte überlegen sein.

Die gestellte Aufgabe wurde dadurch gelöst, indem im Rahmen des erfindungsgemäßen Verfahrens die katalytische Herstellung von gegebenenfalls substituierten aromatischen oder hetereoaromatischen Nitrilen der allgemeinen Formel (I)

Ar-CN (I)

durch Umsetzung der entsprechenden Arylhalogenide der allgemeinen Formel (II),

Ar-X (II)

worin
X für Chlor, Brom, Iod, Triflat (Trifluormethansulfonat), Nonaflat (Nonafluorbutansulfonat), Mesylat oder Tosylat, bevorzugt für Chlor und Brom, besonders bevorzugt für Chlor, und Ar für einen gegebenenfalls substituierten aromatischen oder heteroaromatischen Rest, bevorzugt für einen gegebenenfalls substituierten aromatischen Rest, steht, unter Verwendung von Kaliumhexacyanoferrat(II) (gelbes Blutlaugensalz) gebenenfalls in einem Lösungsmittel und gegebenenfalls in Gegenwart einer Base sowie in Gegenwart einer Pd-Verbindung und n-Butyl-bis(adamantyl)phosphan oder Tri(tert-butyl)phosphan als Phosphanligand.

Als Palladiumverbindungen können bekannte Pd(0)- und Pd(II)-Verbindungen eingesetzt werden. Typische Beispiele sind Salze, wie z.B. PdCl2 und Pd(OAc)2, sowie Pd-Komplexe, wie z.B. Pd(PPh₃)₄, Pd(dba)₂, PdCl₂(PhCN)₂, Allylpalladiumchlorid-Dimer sowie Komplexe von Palladium(0) bzw. (II) mit den oben genannten Liganden der Formeln (III) und (IV). Bevorzugt sind Pd(dba)₂ und Pd(OAc)₂.

Die eingesetzte Palladiumverbindung sollte in einer ausreichenden Menge im Reaktionsgemisch vorhanden sein. Der Fachmann wird die notwendige Einsatzmenge anhand von Wirtschaftlichkeitserwägungen (Schnelligkeit der Reaktion, Ausbeute, Stoffeinsatzkosten) auswählen. Beim erfindungsgemäßen Verfahren können Tumover-Werte der Katalysatoren in der Größenordnung von mindestens 10 bis 100.000 realisiert werden. Vorteilhaft ist der Einsatz der Palladiumverbindung in einer Menge von 0.001 bis 10 mol-% bezogen auf das Aryl- oder Heteroarylhalogenid, bevorzugt werden Mengen von 0.01 mol-% bis 2 mol-% eingesetzt.

Beim eingesetzten Phosphanliganden handelt es sich um n-Butyl-bis(adamantyl)phosphan oder Tri(tert-butyl)phosphan.

Bezüglich der verwendeten Menge des eingesetzten Phosphanliganden können im erfindungsgemäßen Verfahren molare Palladium:Phosphanligand-Verhältnisse von 1:100 bis zu 10:1 verwendet werden. Bevorzugt ist die Verwendung von molaren Palladium:Phosphanligand-Verhältnissen von 1:10 bis 2:1, besonders bevorzugt sind molare Verhältnisse von 1:5 bis 1:1.

Phosphanligand und Palladium können zusammen als Komplex oder einzeln zugesetzt werden.

Als Lösemittel finden bei dem erfindungsgemäßen Verfahren im allgemeinen inerte organische Lösemittel und/oder Wasser Verwendung. Bevorzugt sind dipolar aprotische Lösungsmittel wie z.B. aliphatische Ester oder Amide sowie deren Gemische. Besonders bevorzugt ist die Verwendung von N,N-Dimethylacetamid und N-Methylpyrrolidin-2-on. Die Reaktionen können auch in Substanz, d.h. ohne Lösungsmittel, durchgeführt werden.

Gegebenenfalls kann bei dem erfindungsgemäßen Verfahren der Zusatz von Basen vorteilhaft sein. Dabei können sowohl organische als auch anorganische Basen eingesetzt werden. Beispiele sind Amine, Carboxylate, Carbonate, Hydrogencarbonate, Phosphate, Alkoxide und Hydroxide. Bevorzugt werden Alkali- und Erdalkalimetallacetate, -carbonate und -hydroxide eingesetzt. Besonders bevorzugt ist der Zusatz von Alkalimetallcarbonaten wie Natrium- oder Kaliumcarbonat.

Die eingesetzten Basen werden bevorzugt in Mengen von 1-100 mol-% bezogen auf das Aryl- oder Heteroarylhalogenid verwendet. Besonders bevorzugt sind Mengen von 10 - 50 mol-% der Base.

Als Cyanidquelle wird beim erfindungsgemäßen Verfahren Kaliumhexacyanoferrat(II) eingesetzt. Da im erfindungsgemäßen Verfahren alle sechs Cyanidliganden für die Reaktion zur Verfügung stehen, ist ein Einsatz der Cyanidquelle in einer Menge von 16,7 mol-% oder in einer höheren Menge vorteilhaft. Der Fachmann wird die notwendige Einsatzmenge der Cyanidquelle anhand von Wirtschaftlichkeitserwägungen (Schnelligkeit der Reaktion, Ausbeute, Stoffeinsatzkosten) auswählen. Bevorzugt ist der Einsatz von 15 mol-% bis 50 mol-%, besonders bevorzugt ist der Einsatz von 16 bis 25 mol-% der Cyanidquelle, bezogen auf das Aryl- oder Heteroarylhalogenid.

Die Reaktion wird bei Temperaturen von 20 bis 220°C durchgeführt. Bevorzugt sind Reaktionstemperaturen von 80 bis 200°C, besonders bevorzugt wird bei 100 bis 180°C gearbeitet.

Die Reaktion wird normalerweise drucklos durchgeführt. Sie kann jedoch auch ohne Probleme unter Druck, z.B. in einem Autoklaven oder Druckrohr, durchgeführt werden.

Mit der beim erfindungsgemäßen Verfahren eingesetzten Cyanidquelle Kaliumhexacyanoferrat(II) und dem entsprechenden Katalysatorsystem z.B. aus einer Kombination einer Palladium-Verbindung, eines Phosphanliganden und gegebenenfalls einer Base lassen sich bei Cyanierungen von Aryl- und Heteroarylhalogeniden signifikant bessere Ergebnisse realisieren als mit allgemein bekannten Reaktionssystemen. Gegenüber dem Stand der Technik ist als deutlicher Fortschritt zu sehen, dass es durch das erfindungsgemäße Verfahren ermöglicht wird, unter Verwendung der ungefährlichen und wohlfeilen Blutlaugensalze als Cyanidquelle auch die wenig reaktiven, jedoch sehr kostengünstigen und breiter verfügbaren Chloraromaten zu cyanieren.

Grundsätzlich besteht hinsichtlich des Einsatzes von Aromaten oder Heteroaromaten keine Beschränkung. Insbesondere kann der Rest Ar ein (C₆-C₁₉)-Arylrest bzw. ein (C₃-C₁₈)-Heteroarylrest mit 1, 2 oder 3 Heteroatome wie Stickstoff, Sauerstoff oder Schwefel im Ring sein.

Dabei ist es möglich, dass der Rest Ar bis zu acht Substituenten tragen kann, die unabhängig voneinander (C₁-C₈)-Alkyl, (C₃-C₈)-Cycloalkyl, (C₂-C₈)-Alkenyl, (C₂-C₈)-Alkinyl, (C₇-C₂₀)-Aralkylrest, OH, O-[(C₁-C₈)-Alkyl], OC(O)-[(C₁-C₈)-Alkyl], O-Phenyl, Phenyl, NH₂, NO₂, NO, N[(C₁-C₈)-Alkyl]₂, NH[(C,-C₈)-Alkyl], NHC(O)-[(C₁-C₈)-Alkyl], N[(C₁-C₈)-Alkyl]C(O)-[(C₁-C₈)-Alkyl], SH, S-Phenyl, S-[(C₁-C₈)-Alkyl], Fluor, Chlor, CF₃, CN, COOH, COO-[(C₁-C₈)-Alkyl], CONH-[(C₁-C₈)-Alkyl], COO-Phenyl, CONH-Phenyl, CHO, SO₂-(C₁-C₈)-Alkyl, SO-(C₁-C₈)-Alkyl, PO-(Phenyl)₂, PO-[(C₁-C₈)-Alkyl]₂, PO₃H₂, PO[O-(C₁-C₈)-Alkyl]₂, SO₃H, SO₃M, SO₃-[(C₁-C₈)-Alkyl], Si[(C₁-C₈)-Alkyl]₃, (C₁-C₈)-Haloalkyl sowie (C₁-C₈)-Acyl sein können.

Als (C₁-C₈)-Alkyl sind anzusehen Methyl, Ethyl, n-Propyl, Isopropyl, n-Butyl, Isobutyl, sec-Butyl, tert-Butyl, Pentyl, Hexyl, Heptyl oder Octyl samt aller Bindungsisomeren. Diese können einfach oder mehrfach mit (C₁-C₈)-Alkoxy, (C₁-C₈)-Haloalkyl, OH, Halogen, NH₂, NO₂, SH, S-(C₁-C₈)-Alkyl substituiert sein.

Als (C₂-C₈)-Alkenyl ist mit Ausnahme von Methyl ein wie oben dargestellter (C₁-C₈)-Alkyl-Rest zu verstehen, der mindestens eine Doppelbindung aufweist.

Unter (C₂-C₈)-Alkinyl ist mit Ausnahme von Methyl ein wie oben dargestellter (C₁-C₈)-Alkyl-Rest zu verstehen, der mindestens eine Dreifachbindung aufweist.

Unter (C₁-C₈)-Acyl versteht man einen über eine -C=O-Funktion ans Molekül gebundenen (C₁-C₈)-Alkyl-Rest.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-atomhaltige Reste substituiert sein und/oder N-, O-, P-, S-Atome im Ring aufweisen, z. B. 1-, 2-, 3-, 4-Piperidyl, 1-, 2-, 3-Pyrrolidinyl, 2-, 3-Tetrahydrofuryl, 2-, 3-, 4-Morpholinyl. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Unter einem (C₆-C₁₉)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste. Dieser kann einfach oder mehrfach mit (C₁-C₈)-Alkoxy, (C₁-C₈)-Haloalkyl, OH, Halogen, NH₂, NO₂, SH, S-(C₁-C₈)-Alkyl, (C₁-C₈)-Acyl, (C₁-C₈)-Alkyl substituiert sein.

Ein (C₇-C₂₀)-Aralkylrest ist ein über einen (C₁-C₈)-Alkylrest an das Molekül gebundener (C₆-C₁₉)-Arylrest.

(C₁-C₈)-Alkoxy ist ein über ein Sauerstoffatom an das betrachtete Molekül gebundener (C₁-C₈)-Alkyl-Rest.

(C₁-C₈)-Haloalkyl ist ein mit einem oder mehreren Halogenatomen substituierter (C₁-C₈)-AlkylRest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches 1, 2 oder 3 Heteroatome wie Stickstoff, Sauerstoff oder Schwefel im Ring aufweist. Als solche Heteroaromaten werden insbesondere Reste angesehen wie 1-, 2-, 3-Furyl, 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imjdazolyl, Acridinyl, Chinolinyl, Phenanthridinyl, 2-, 4-, 5-, 6-Pyrimidinyl. Unter einem (C₄-C₁₉)-Heteroaralkyl wird ein dem (C₇-C₂₀)-Aralkylrest entsprechendes heteroaromatisches System verstanden.

Als Halogene kommen Fluor, Chlor, Brom und Iod in Frage.

### Beispiele

Allgemeine Arbeitsvorschrift für die folgenden Beispiele:

0,4 mmol Natriumcarbonat, 0,4 mmol Gelbes Blutlaugensalz, eine geeignete Menge Palladiumacetat und Ligand wurden, wie in der Tabelle angegeben, in einem Druckrohr unter Argon in 2 ml trockenem NMP suspendiert. Es wurden 2 mmol Halogenaromat und 200 ml Hexadecan als interner Standard für die GC-Analytik zugegeben. Das Druckrohr wurde verschlossen und 16 h auf die angegebene Temperatur geheizt. Nach Abkühlen auf Raumtemperatur wurde mit 2 ml Wasser und 2 ml Ether verdünnt. Eine Probe der organischen Phase wurde gaschromatographisch untersucht. Zur Produktisolierung wurde die wässige Phase mit Ether extrahiert. Die vereinigten organischen Phasen wurden mit Wasser und gesättigter NaCl-Lösung gewaschen und schließlich über Natriumsulfat getrocknet. Nach Abziehen des Lösungsmittels wurde das Rohprodukt durch Säulenchromatographie über Kieselgel oder destillativ gereinigt.

| **Nr.** | **ArX** | **Pd(OAc)₂** | **Ligand** | **Temp.** | **Ausbeute** | **TON** |
|---|---|---|---|---|---|---|
| | | **[mol%]** | **(mol%)** | **[°C]** | **[%]** | |
| 1 | Chlorbenzol | 0,1 | BuPAd₂ (0,3) | 140 | 100 | 1000 |
| 2 | Chlorbenzol | 0,1 | PtBu₃ (0,3) | 140 | 88 | 880 |
| 3 | 2-Chlortoluol | 0,1 | BuPAd₂ (0,3) | 160 | 90 | 900 |
| 4 | 4-Chlortoluol | 0,2 | BuPAd₂ (0,6) | 160 | 80 | 400 |
| 5 | 2-Chlor-m-xylol | 0,5 | BuPAd₂ (1) | 140 | 69 | 138 |
| 6 | 3-Chlorpyridin | 0,25 | BuPAd₂ (1) | 160 | 95 | 380 |
| 7 | Brombenzol | 0,05 | BuPAd₂ (0,2) | 140 | 100 | 2000 |
| 8 | 2-Bromtoluol | 0,05 | BuPAd₂ (0,2) | 140 | 100 | 2000 |
| 9 | 1-Bromnaphthalin | 0,05 | BuPAd₂ (0,2) | 140 | 100 | 2000 |

## Patentansprüche

1. Verfahren zur katalytischen Herstellung von gegebenenfalls substituierten aromatischen oder hetereoaromatischen Nitrilen der allgemeinen Formel (I)
Ar-CN (I)
durch Umsetzung der entsprechenden Arylhalogenide der allgemeinen Formel (II),
Ar-X (II)
worin
X für Chlor, Brom, Iod, Triflat, Nonaflat, Mesylat oder Tosylat und
Ar für einen gegebenenfalls substituierten aromatischen oder heteroaromatischen Rest steht,
**dadurch gekennzeichnet, dass** die Umsetzung in Gegenwart einer Palladium-Verbindung, n-Butyl-bis(adamantyl)phosphan oder Tri(tert-butyl)phosphan und Kaliumhexacyanoferrat (II) gegebenenfalls in einem Lösungsmittel und gegebenenfalls unter Hinzufügen einer Base, durchgeführt wird.

2. Verfahren nach Anspruch 1, **dadurch gekennzeichnet, dass** als Palladiumverbindungen bekannte Palladium(0)-Komplexe oder Palladium(II)-Salze oder -Komplexe eingesetzt werden.

3. Verfahren nach Anspruch 1 und 2, **dadurch gekennzeichnet, dass** man als Palladiumverbindungen Palladiumhalogenide und deren Komplexe, Palladiumacetat, Palladiumdibenzylidenaceton-Komplexe oder Allylpalladiumchlorid-Dimer einsetzt.

4. Verfahren nach einem der Ansprüche 1 bis 3, **dadurch gekennzeichnet, dass** man die Palldiumverbindung in einer Menge von 0.001 mol-% bis 10 mol-% bezogen auf das eingesetzte Arylhalogenid Ar-X, verwendet.

5. Verfahren nach einem der Ansprüche 1 bis 4, **dadurch gekennzeichnet, dass** die Umsetzung bei Temperaturen von 20 bis 220°C erfolgt.

6. Verfahren nach einem der Ansprüche 1 bis 5, **dadurch gekennzeichnet, dass** die Reaktion drucklos durchgeführt wird.

7. Verfahren nach einem der Ansprüche 1 bis 6, **dadurch gekennzeichnet, dass** Ar ein (C₆-C₁₉)-Arylrest oder ein (C₃-C₁₈)-Heteroarylrest mit 1, 2 oder 3 Heteroatomen ausgewählt aus Stickstoff, Sauerstoff oder Schwefel, im Ring ist.

8. Verfahren nach einem der Ansprüche 1 bis 7, **dadurch gekennzeichnet, dass** A in Formel (IV) eine Alkyleneinheit mit 1 bis 10 C-Atomen ist.

## Claims

1. Process for catalytically preparing optionally substituted aromatic or heteroaromatic nitriles of the general formula (I)
Ar-CN (I)
by reacting the corresponding aryl halides of the general formula (II)
Ar-X (II)
in which
X is chlorine, bromine, iodine, triflate, nonaflate, mesylate or tosylate and
Ar is an optionally substituted aromatic or heteroaromatic radical,
**characterized in that** the reaction is performed in the presence of a palladium compound, n-butylbis(adamantyl)phosphine or tri(tert-butyl)phosphine and potassium hexacyanoferrate(II), optionally in a solvent and optionally with addition of a base.

2. Process according to Claim 1, **characterized in that** the palladium compounds used are known palladium(0) complexes or palladium(II) salts or complexes.

3. Process according to Claim 1 and 2, **characterized in that** the palladium compounds used are palladium halides and complexes thereof, palladiumacetate, palladium-dibenzylideneacetone complexes or allylpalladium chloride dimer.

4. Process according to one of Claims 1 to 3, **characterized in that** the palladium compound is used in an amount of 0.001 mol% to 10 mol% based on the aryl halide Ar-X used.

5. Process according to one of Claims 1 to 4, **characterized in that** the reaction is effected at temperatures of 20 to 220°C.

6. Process according to one of Claims 1 to 5, **characterized in that** the reaction is performed at ambient pressure.

7. Process according to one of Claims 1 to 6, **characterized in that** Ar is a (C₆-C₁₉)-aryl radical or a (C₃-C₁₈)-heteroaryl radical with 1, 2 or 3 heteroatoms selected from nitrogen, oxygen and sulphur in the ring.

8. Process according to one of Claims 1 to 7, **characterized in that** A in formula (IV) is an alkylene unit having 1 to 10 carbon atoms.

## Revendications

1. Procédé pour la production catalytique de nitriles aromatiques ou hétéroaromatiques éventuellement substitués de formule générale (I)
Ar-CN (I)
par mise en réaction des halogénures d'aryle correspondants de formule générale (II),
Ar-X (II)
formules dans lesquelles
X représente le chlore, le brome, l'iode, le triflate, nonaflate, mésylate ou tosylate et
Ar représente un radical aromatique ou hétéroaromatique éventuellement substitué,
**caractérisé en ce que** la réaction est effectuée en présence d'un composé contenant du palladium, de n-butyl-bis(adamantyl)phosphane ou de tri(tert-butyl)-phosphane et d'hexacyanoferrate de potassium (II) éventuellement dans un solvant et éventuellement avec addition d'une base.

2. Procédé selon la revendication 1, **caractérisé en ce qu'**on utilise comme composés contenant du palladium des complexes de palladium(0) ou des sels ou complexes de palladium(II) connus.

3. Procédé selon la revendication 1 et 2, **caractérisé en ce qu'**on utilise comme composés contenant du palladium des halogénures de palladium et leurs complexes, l'acétate de palladium, des complexes palladium-dibenzylidène-acétone ou le dimère de chlorure d'allylpalladium.

4. Procédé selon l'une quelconque des revendications 1 à 3, **caractérisé en ce qu'**on utilise le composé contenant du palladium en une quantité de 0,001 % en moles à 10 % en moles par rapport à l'halogénure d'aryle Ar-X utilisé.

5. Procédé selon l'une quelconque des revendications 1 à 4, **caractérisé en ce que** la réaction s'effectue à des températures de 20 à 220 °C.

6. Procédé selon l'une quelconque des revendications 1 à 5, **caractérisé en ce que** la réaction est effectuée sans pression.

7. Procédé selon l'une quelconque des revendications 1 à 6, **caractérisé en ce que** Ar est un radical aryle en C₆-C₁₉ ou un radical hétéroaryle en C₃-C₁₈ comportant dans le cycle 1, 2 ou 3 hétéroatomes choisis parmi les atomes d'azote, d'oxygène et de soufre.

8. Procédé selon l'une quelconque des revendications 1 à 7, **caractérisé en ce que** A dans la formule (IV) est une unité alkylène ayant de 1 à 10 atomes de carbone.
